# EUROPEAN PATENT SPECIFICATION

(11) **EP 3 209 254 B1**
(45) Date of publication and mention of the grant of the patent: **29.08.2018**
(21) Application number: 15785185.8
(22) Date of filing: 24.09.2015
(51) Int. Cl.: A61F 9/013, A61B 17/3211

(54) **CHOPPER, FOR CATARACT OPERATIONS**
CHOPPER FÜR KATARAKTCHIRURGIE
DISPOSITIF DE COUPE POUR LES OPÉRATIONS DE LA CATARACTE

(30) Priority: 20.10.2014 IT RM20140593
(43) Date of publication of application: 30.08.2017
(73) Proprietor: Pentalfa Solutions S.r.l., 80131 Napoli (NA) (IT)
(72) Inventor: CARBONARA, Claudio, 80131 Napoli (NA) (IT)
(74) Representative: Fiammenghi, Eva
(86) International application number: PCT/IB2015/057350
(87) International publication number: WO 2016/063153

(56) References cited:
- US-A1- 2006 036 270

## Description

### Field of the art

The present invention refers to the ophthalmic surgery instruments and in particular to a so-called "chopper" used in surgical cataract operations.

### Prior art

It is known that the cataract constitutes a rather widespread problem, above all in elderly people 65 years old and up, and consists of the opacification of the crystalline lens following oxidative processes of the material that forms the crystalline lens itself. In the cataract operation, substantially, the crystalline lens of the eye is substituted with an appropriate artificial crystalline lens adapted for the characteristics of the single patient. Today, increasingly sophisticated techniques are applied for reducing the post-operative problems, above all by reducing the size of the cornea incision.

One instrument used in this operation type is the so-called chopper.

The chopper comprises a handle (preferably with at least partly knurled side surface) which is held by the surgeon's hand during the operation, such handle having a needle-shaped head at its distal end. Such needle-shaped head, or "needle-head", has a first rectilinear section coaxial with respect to the geometric axis of the handle, which then flows into a second section, also rectilinear. The first section and the second section of the needle-head form a sharp angle between them that in the conventional choppers is equal to about 135°. The distal end of the second section of the needle-head, or needle-like head, of the chopper, bears the cutting part that serves for cutting the tissue of the eye.

Said cutting part forms a right angle with respect to the second section of the needle-head and is bent in the opposite direction with respect to the angle formed between the first and the second section. In other words, the right angle is situated on the "opposite side" of the needle-head with respect to the angle of about 135° formed between the first section and the second section, and the cutting part (which could be defined "third section" or "final section" of the needle-head) is situated on the same geometric plane of the first section and second section of the needle-head; this signifies that all the rectilinear sections or portions of the needle-head lie in a single geometric plane.

The geometric form of the third section, or cutting part, which substantially constitutes the "active" free end of the surgical instrument, can vary from one instrument to another. The present invention first of all regards the surgical instruments called "choppers" whose cutting part is triangular prismatic. Nevertheless, the present invention is not necessarily limited to such prismatic form of the cutting part.

The needle-head has a cross section which is thinned towards its free end, i.e. progressively as one moves away from the handle of the chopper itself. In the conventional instrument, the cutter of the cutting part is substantially directed towards the handle of the chopper.

With regard to the size, the chopper can have a handle of about 9 cm length, a first section of about 1.5 cm length, a second section of about 1.3 cm length, and a third section (cutting part) of about 2 mm length. For children, cutting parts are used of length variable between about 2 mm and about 1 mm.

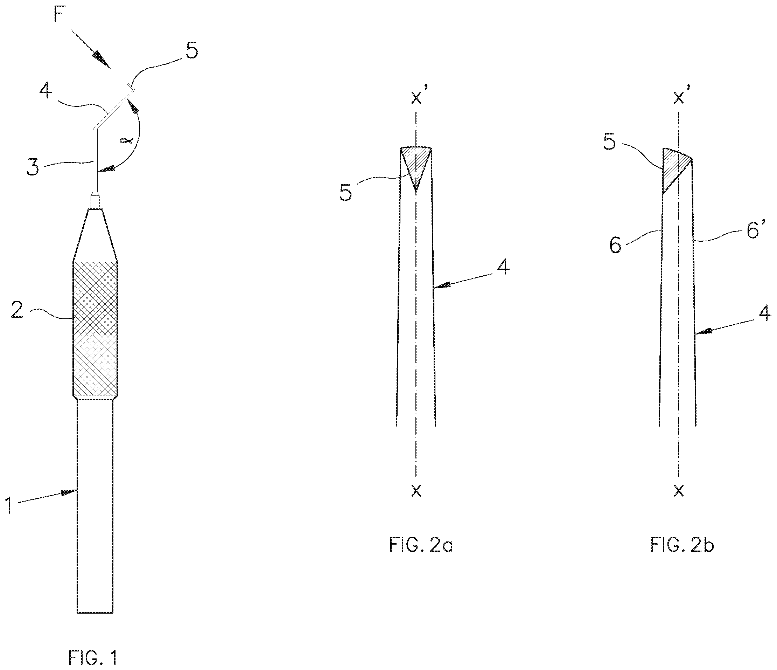

In figure 1 of the drawings enclosed with the present document, as in figures 2a and 2b (details relative to the cutting tip of the chopper), a conventional chopper with prismatic cutting tip is shown.

In Fig. 1, reference number 1 indicates the entire handle of the chopper, 2 specifically indicates the knurled portion of the handle 1 that serves to increase the grip of the surgeon's hand on the handle 1, 3 indicates the first section of the needle head, 4 the second section bent at 135° with respect to the first section, and finally 5 the cutting tip ("active part") is shown which is bent 90° with respect to the second section 4 of the needle-head, in the opposite direction with respect to the bend direction of the second section 4 with respect to the first section 3.

The subsequent figures, i.e. Figs. 2a and 2b, are views in the direction of the arrow F in Fig. 1, i.e. they are plan views on a plane orthogonal to the plane of the sheet (plane of the drawing) of Fig. 1, in which such plane passes through the longitudinal geometric axis (X-X') of the second section 4. The geometric axis X-X' is clearly illustrated in Figs. 2a and 2b. In other words, by using an optical instrument with a suitable magnification, one can observe that the cutting tip 5 in the conventional choppers assumes the form of a triangle (in the view in the direction of said arrow F). The vertex of such isosceles triangle shown with a shading in figures 2a and 2b, respectively, never "exits" from the shape, i.e. from the profile of the second section 4 of the needle head. In the first case (Fig.2a), the cutter, i.e. the tip of the shaded triangle, or better yet the respective geometric projection, is situated along the longitudinal symmetry axis X-X' of the second section 4 of the needle head, while in the second case (Fig. 2b) the tip of the shaded triangle (or better yet its projection on the plane of the drawing) lies on the edge of the second section 4 and one side of the isosceles triangle is aligned with respect to said edge of the second section 4 of the needle-head.

In other words, this signifies that with respect to the longitudinal symmetry axis X-X' of the second section 4 of the needle-head, the triangular prismatic body constituting the cutting tip 5 can at most be rotated, around its own axis (axis orthogonal to the plane of the sheet of Fig. 2a and 2b), of an angle such to align one side of the shaded isosceles triangle with one edge 6 of the same second section 4. Quite simply, in the prior art the isosceles triangle never exits - in its geometric projection - from the profile or shape of said second section 4 of the needle of the chopper. Figures 2a and 2b show the two "extreme cases" allowed in the prior art, with regard to a rotation direction of the cutting tip 5; obviously the tip could also be rotated in the direction opposite its own axis, in a manner such to be able to align the other side of the isosceles triangle with the lateral opposite edge 6' of the profile of the second section 4. In each case, whether the tip 5 is rotated in one direction or in the opposite direction, in the conventional technique the corresponding rotation angle is small, so that the isosceles triangle shown with shading in Fig. 2a and Fig. 2b never "exits" from the profile of the second section 4 of the needle-head.

This type of conventional chopper encounters the following problem.

If the patient has a rather large nose and/or has a sunken eye, the inventor has observed that the surgical operation (with regard to the use of the chopper) is made more difficult. Specifically, if the surgeon is right-handed, during the operation he must rotate the hand in one direction and then assume an unnatural position during the execution of the incision. This rotation is also made necessary by the fact that the angle "α" of 135° between the two sections 3 and 4 of the needle-head (see Fig. 1) is unsuitable for the considered case (large nose and/or has a sunken eye). Now, in the general medical field as well as in the specific field that regards the present invention, the importance of reducing the fatigue and stress for the surgeon during the operation is well-known. This ensures great concentration for the surgeon and allows minimizing the risks for the patient, i.e. the errors by the surgeon. Hence, one object of the present invention is to provide a chopper that optimizes its ease of use by the surgeon during the cataract operation.

A further object is to not increase the production costs, by making only slight but substantial modifications to the conventional choppers. In other words the materials can remain the same (those conventionally used in the field) and also the production process substantially does not undergo important modifications.

A final consideration regards the form of the needle-head. As can be inferred from the drawing, this is not only bent but tapered, i.e. normally the diameter of the cross section of the needle constantly decreases towards the free end of the cutting tip 5, and this also holds true for the same cutting tip 5. This signifies that if the cutting tip 5 is prismatic, one triangular base thereof (the free base) has an area slightly less than that of the other base (placed on the side of the second section 4). Of course, these details are not illustrated in figure 2a and in figure 2b so to not overly complicate the drawing and the relative interpretation.

### Description of the invention

The present invention solves the preceding problems by providing a chopper for cataract operations as defined in the first claim. The dependent claims regard several embodiment variants of the invention.

US-A-2006/036270 (D1) discloses all the subject matter of the preamble of claim 1.

### Brief description of the drawings

The present invention will now be illustrated with reference to the enclosed figures, which show a particular non-limiting embodiment thereof.

The figures show:
FIGURE 1 - a conventional chopper, by way of comparison;
FIGURE 2 (a, b) - the details relative to the cutting tip of the chopper of figure 1, in two embodiment variants;
FIGURE 3 - the chopper of the present invention;
FIGURE 4 (a, b) - the details relative to the cutting tip of the chopper of figure 3, in two embodiment variants, for embodiment variants, respectively for right-handed surgeons (Fig.
4a) and for left-handed surgeon (Fig. 4b).

### Detailed description of a preferred embodiment of the invention

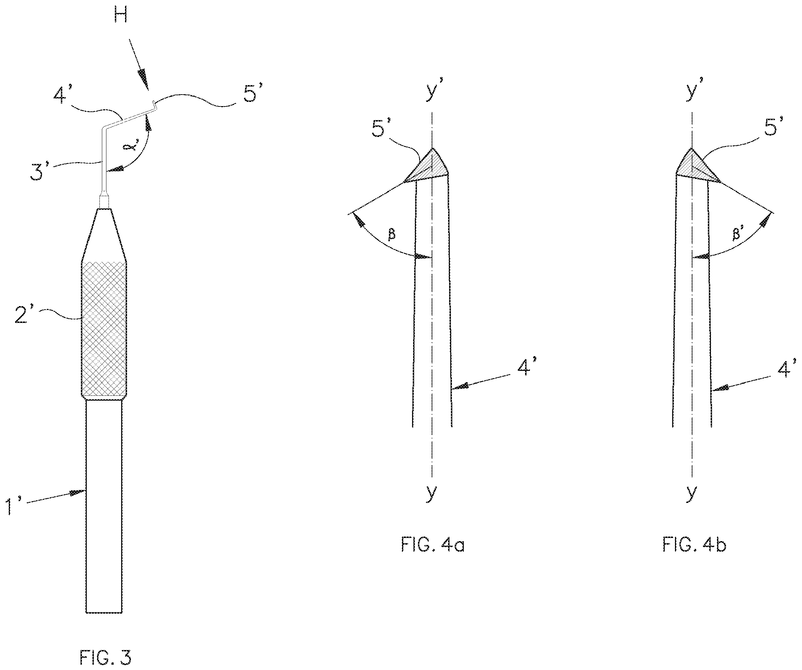

The present invention will now be described as a mere non-limiting example, by making reference to figures 3, 4a and 4b.

First of all, it is observed in figure 3 that the angle (α') between the first section 3' and the second section 4', indicated herein with the primes in order to distinguish the chopper of the invention from the conventional chopper shown in the preceding figures, is less than the angle (α) defined between the corresponding elements 3 and 4 of the conventional needle-head of Fig. 1. Specifically, the angle (α') between the first section 3' and the second section 4' of the chopper according to the present invention is equal, in this embodiment, to about 110°. According to the present invention, it generally holds true that said angle (α') between the first section 3' and the second section 4' of the needle-head is comprised between about 110° and about 135°, but preferably such angle is less than about 120° and still more preferably it is equal to about 110°. Hence, according to the present invention, the inventor has found that by selecting an angle less than 135° and in particular less than 120°, and better yet slightly greater than 110°, the operation can be conducted without overly rotating the hand and hence with greater comfort.

In addition by observing in the direction of the arrow H of figure 3, the triangular prismatic tip 5' (in this embodiment) of the chopper is rotated in clockwise sense by an angle (β) of about 60° with respect to the longitudinal symmetry axis (Y-Y') of the second section 4' of the chopper of the present invention. This configuration of the needle-head (shown in Fig. 4a) is that which will be used by right-handed surgeons. Analogously, the rotation of the cutting tip 5' will be equal and opposite in the case of a left-handed surgeon, who will use a chopper having a prismatic tip "rotated" in counterclockwise sense by an angle (β') equal to about 60° with respect to the longitudinal symmetry axis Y-Y' of the rectilinear second section 4' of the chopper 1'.

In general said angles (β, β') of opposite algebraic sign, have an absolute value comprised between about 45° and about 70°, preferably between about 55° and 65°, and still more preferably they are equal to about 60° (optimal value).

A right-handed surgeon who uses a chopper like that indicated in Fig. 3 and Fig. 4a can operate with greater comfort, without having to excessively rotate the hand in case of particular patients (large nose and/or sunken eyes), with respect to the case of the conventional chopper of Fig. 1. Similarly, a left-handed surgeon will be benefited by using a chopper like that shown in Fig. 3 and Fig. 4b, for the same reasons.

Hence, the surgeon will operate in a more natural hand position, for all the different facial anatomical forms of the patient.

Naturally, also in the case of the present invention, the section of the needle-head tends to be thinned towards its free end, including the cutting tip 5' (as is also inferred from figure 3). Nevertheless, the thinning of the same triangular prismatic tip 5' towards its free end is not shown in Figs. 4a and 4b, so as to not overly complicate the interpretation of the drawing, even if this is obvious for a man skilled in the art.

The present invention therefore solves the above-illustrated problems without having to modify the conventional materials and the general form of the chopper, by only operating on the working of the needle-head, in its specific points, and hence substantially without altering the production costs.

Finally, it is observed that in the figures, the first rectilinear section 3' and the second rectilinear section 4' of the needle-head form a sharp angle between them. Of course, this does not remove the fact that of course such angle could also be slightly rounded in other embodiments (in this case the two rectilinear sections would form a kind of "asymptote"). In addition, the form of the cutting part might also not be prismatic, but rather assume other known forms.

## Claims

1. Chopper, for ophthalmic surgery and in particular for cataract operations, comprising a grip handle (1') and a needle-like head, or needle-head, mounted in a fixed or interchangeable manner at a distal end of the grip handle (1'), said needle-head comprising a needle having a first rectilinear section (3') substantially coaxial with a geometric symmetry axis of the grip handle (1') and a substantially rectilinear second section (4') that forms a first angle α' with the first section (3'), and finally a distal cutting part (5'), for cutting the tissues of the patient's eye, said cutting part (5') forming a right angle with respect to the second section (4') with opposite bend direction with respect to said first angle α' and having a length lower than that of the preceding sections (3', 4') of the needle-head, said cutting part (5') and said first and second section (3', 4') of the needle-head all being coplanar, and the cutting part (5') having a cutter with cusp form, in cross section; said cutter or cusp of the cutting part (5') is rotated a second angle β or β' around a longitudinal axis of the cutting part (5'), said second angle β or β' having absolute value greater than 45° and less than 70°, taking as null rotation reference β = 0 or β'=0 the longitudinal geometric axis Y-Y' of said second section (4') of the needle-head, the chopper being **characterized in that** said first angle α' is less than 135° and greater than or equal to 110°.

2. Chopper according to claim 1, **characterized in that** said first angle α' is less than 120°.

3. Chopper according to claim 2, **characterized in that** said first angle α' is equal to 110°.

4. Chopper according to any one of the preceding claims, **characterized in that** said second angle β or β' is comprised, in absolute value, between 55° and 65°.

5. Chopper according to claim 4, **characterized in that** said second angle β or β' is equal to 60° in absolute value.

6. Chopper according to any one of the preceding claims, **characterized in that** for an observer who observes the cutting tip (5') from a direction H, coinciding with the longitudinal geometric axis of the cutting tip (5'), said second angle is negative, β in clockwise sense, if the chopper is used by a right-handed surgeon and it is positive, β' in anticlockwise sense, if the chopper is used by a left-handed surgeon.

7. Chopper according to any one of the preceding claims, **characterized in that** the cutting tip (5') has a prismatic form with isosceles triangle cross section.

8. Chopper according to any one of the preceding claims, **characterized in that** the needle of said needle-head has a cross section with area decreasing starting from the substantially rectilinear first section (3') up to the free end of said cutting part (5').

9. Chopper according to any one of the preceding claims, **characterized in that** said first angle α' defines a sharp edge or angle between the first section (3') and the second section (4').

10. Chopper according to any one of the preceding claims, **characterized in that** said grip handle (1') has a length of about 9 cm, said first section (3') has a length of 1.5 cm, and said second section (4') has a length of 1.2 cm.

11. Chopper according to any one of the preceding claims, **characterized in that** the length of the cutting part (5') is comprised between 1 mm and 2 mm.

## Patentansprüche

1. Chopper für Augenchirurgie und insbesondere für Kataraktoperationen, der einen Handgriff (1') und einen nadelförmigen Kopf oder einen Nadelkopf aufweist, der auf feste oder austauschbare Weise an einem distalen Ende des Handgriffs (1') befestigt ist, wobei der genannte Nadelkopf eine Nadel mit einem ersten geradlinigen Abschnitt (3'), der im Wesentlichen koaxial zu einer geometrischen Symmetrieachse des Handgriffs (1') ist, und einen im Wesentlichen geradlinigen zweiten Abschnitt (4'), der mit dem ersten Abschnitt (3') einen ersten Winkel α' bildet, und schließlich einen distalen Schneideteil (5'), um das Gewebe des Auges des Patienten zu schneiden, aufweist, wobei der genannte Schneideteil (5') bezüglich des zweiten Abschnitts (4') einen rechten Winkel mit im Hinblick auf den ersten Winkeln α' entgegengesetztem Biegewinkel bildet und eine geringere Länge als die vorangehenden Abschnitte (3', 4') des Nadelkopfs aufweist, wobei der genannte Schneideteil (5') und der genannte erste und zweite Abschnitt (3', 4') des Nadelkopfs alle koplanar sind und wobei der Schneideteil (5') eine Schneideeinrichtung mit im Querschnitt spitzenförmiger Gestalt aufweist; wobei die Schneideeinrichtung oder die Spitze des Schneideteils (5') um einen zweiten Winkel β oder β' um eine Längsachse des Schneideteils (5') herum gedreht ist, wobei der genannte zweite Winkel β oder β' einen Absolutbetrag von mehr als 45° und weniger als 70° aufweist, wobei als Referenz für eine Nulldrehung β = 0 oder β' = 0 die geometrische Längsachse Y-Y' des genannten zweiten Abschnitts (4') des Nadelkopfs verwendet wird, wobei der Chopper **dadurch gekennzeichnet ist, dass** der genannte erste Winkel α' kleiner als 135° und größer oder gleich 110° ist.

2. Chopper gemäß Anspruch 1, **dadurch gekennzeichnet, dass** der genannte erste Winkel α' kleiner als 120° ist.

3. Chopper gemäß Anspruch 2, **dadurch gekennzeichnet, dass** der genannte erste Winkel α' gleich 110° ist.

4. Chopper gemäß einem der vorangehenden Ansprüche, **dadurch gekennzeichnet, dass** der genannte zweite Winkel β oder β', als absoluter Wert, zwischen 55° und 65° aufweist.

5. Chopper gemäß Anspruch 4, **dadurch gekennzeichnet, dass** der genannte zweite Winkel β oder β', als absoluter Wert, gleich 60° ist.

6. Chopper gemäß einem der vorangehenden Ansprüche, **dadurch gekennzeichnet, dass** der genannte zweite Winkel für einen Beobachter, der die Schneidspitze (5') von einer Richtung H, die mit der geometrischen Längsachse der Schneidspitze (5') zusammenfällt, aus beobachtet, mit β im Uhrzeigersinn, negativ ist, wenn der Chopper von einem rechtshändigen Operateur benutzt wird, und, mit β' im Gegenuhrzeigersinn, positiv ist, wenn der Chopper von einem linkshändigen Operateur benutzt wird.

7. Chopper gemäß einem der vorangehenden Ansprüche, **dadurch gekennzeichnet, dass** die Schneidspitze (5') eine prismatische Gestalt einem Querschnitt eines gleichschenkligen Dreiecks aufweist.

8. Chopper gemäß einem der vorangehenden Ansprüche, **dadurch gekennzeichnet, dass** die Nadel des genannten Nadelkopfs einen Querschnitt mit einer Fläche, die sich beginnend von dem im Wesentlichen geradlinigen ersten Abschnitt (3') bis zu dem freien Ende des genannten Schneidteils (5') verringert, aufweist.

9. Chopper gemäß einem der vorangehenden Ansprüche, **dadurch gekennzeichnet, dass** der genannte erste Winkel α' eine(n) scharfe Kante oder Winkel zwischen dem ersten Abschnitt (3') und dem zweiten Abschnitt (4') definiert.

10. Chopper gemäß einem der vorangehenden Ansprüche, **dadurch gekennzeichnet, dass** der genannte Handgriff (1') eine Länge von etwa 9 cm aufweist, wobei der genannte erste Abschnitt (3') eine Länge von 1,5 cm aufweist und wobei der genannte zweite Abschnitt (4') eine Länge von 1,2 cm aufweist.

11. Chopper gemäß einem der vorangehenden Ansprüche, **dadurch gekennzeichnet, dass** die Länge des Schneideteils (5') zwischen 1 mm und 2 mm beträgt.

## Revendications

1. Chopper pour la chirurgie ophtalmologique et en particulier pour les opérations de la cataracte, comprenant une poignée (1') et une tête en forme d'aiguille, ou tête à aiguille, montée de manière fixe ou interchangeable sur une extrémité distale de la poignée (1'), ladite tête à aiguille comprenant une aiguille qui présente une première section rectiligne (3') globalement coaxiale par rapport à un axe de symétrie géométrique de la poignée (1'), et une seconde section (4') globalement rectiligne qui forme un premier angle α' avec la première section (3'), et enfin une partie de coupe distale (5') pour couper les tissues de l'oeil du patient, ladite partie de coupe (5') formant un angle droit par rapport à la seconde section (4), avec une direction de coude opposée par rapport au premier angle α', et présentant une longueur inférieure à celle des sections précédentes (3', 4') de la tête à aiguille, la partie de coupe (5') et les première et seconde sections (3', 4') de la tête à aiguille étant toutes coplanaires, et la partie de coupe (5') comportant un organe de coupe en forme de pointe, en coupe transversale ; l'organe de coupe ou pointe de la partie de coupe (5') étant tourné d'un second angle β ou β' autour d'un axe longitudinal de la partie de coupe (5'), ledit second angle β ou β' présentant une valeur absolue supérieure à 45° et inférieure à 70°, avec comme référence de rotation nulle β=0 ou β'=0 l'axe géométrique longitudinal Y-Y' de la seconde section (4') de la tête à aiguille, le chopper étant **caractérisé en ce que** le premier angle α' est inférieur à 135° et supérieur ou égal à 110°.

2. Chopper selon la revendication 1, **caractérisé en ce que** le premier angle α' est inférieur à 120°.

3. Chopper selon la revendication 2, **caractérisé en ce que** le premier angle α' est égal à 110°.

4. Chopper selon l'une quelconque des revendications précédentes, **caractérisé en ce que** le second angle β ou β' est compris, en valeur absolue, entre 55° et 65°.

5. Chopper selon la revendication 4, **caractérisé en ce que** le second angle β ou β' est égal à 60° en valeur absolue.

6. Chopper selon l'une quelconque des revendications précédentes, **caractérisé en ce que** pour un observateur qui observe la pointe de coupe (5') à partir d'une direction H qui coïncide avec l'axe géométrique longitudinal de la pointe de coupe (5'), le second angle est négatif, β dans le sens des aiguilles d'une montre, si le chopper est utilisé par un chirurgien droitier, et il est positif, β' dans le sens inverse des aiguilles d'une montre, si le chopper est utilisé par un chirurgien gaucher.

7. Chopper selon l'une quelconque des revendications précédentes, **caractérisé en ce que** l'extrémité de coupe (5') a une forme prismatique avec une section transversale en forme de triangle isocèle.

8. Chopper selon l'une quelconque des revendications précédentes, **caractérisé en ce que** l'aiguille de la tête à aiguille a une section transversale avec une superficie qui va en diminuant, à partir de la première section globalement rectiligne (3'), jusqu'à l'extrémité libre de ladite partie de coupe (5').

9. Chopper selon l'une quelconque des revendications précédentes, **caractérisé en ce que** le premier angle α' définit une arête vive ou un angle saillant entre la première section (3') et la seconde section (4').

10. Chopper selon l'une quelconque des revendications précédentes, **caractérisé en ce que** la poignée (1') a une longueur d'environ 9 cm, la première section (3') a une longueur de 1,5 cm, et la seconde section (4') a une longueur de 1,2 cm.

11. Chopper selon l'une quelconque des revendications précédentes, **caractérisé en ce que** la longueur de la partie de coupe (5') est comprise entre 1 mm et 2 mm.
